# FASCICULE DE BREVET EUROPEEN

(11) **EP 1 883 486 B1**
(45) Date de publication et mention de la délivrance du brevet: **19.12.2012**
(21) Numéro de dépôt: 06764682.8
(22) Date de dépôt: 24.05.2006
(51) Int. Cl.: B23B 31/12

(54) **Mandrin porte-outil pour l'équipement d'une machine tournante, notamment du type clé à choc**
Werkzeughalterfutter für eine Rotationsmaschine, im Besonderen für einen Schlagschrauber
Tool-holder chuck for equipping a rotating machine, in particular of the impact wrench type

(30) Priorité: 27.05.2005 FR 0505389
(43) Date de publication de la demande: 06.02.2008
(73) Titulaire: ETABLISSEMENTS AMYOT S.A., F-25300 Pontarlier (FR)
(72) Inventeur: CACHOD, Yves, F-25270 Levier (FR)
(74) Mandataire: Maureau, Philippe
(86) Numéro de dépôt international: PCT/FR2006/001190
(87) Numéro de publication internationale: WO 2006/125918

(56) Documents cités:
- EP-A- 1 293 289
- EP-A1- 0 461 806
- DE-C1- 4 238 464
- DE-C1- 4 313 742
- SU-A1- 1 590 210
- US-A- 2 855 207
- US-A- 4 423 881

## Description

La présente invention concerne un mandrin porte-outil pour l'équipement d'une machine tournante, notamment du type « clé à choc ».

Un mandrin monté sur l'arbre d'une machine tournante vise à réaliser la fixation d'un outil, tel qu'un foret s'il s'agit d'un outil de perçage. La fixation de l'outil sur le mandrin est couramment réalisée par l'intermédiaire de trois mors convergeant vers l'avant, entraînés et guidés par différents moyens ménagés dans le mandrin, de telle sorte que le déplacement axial des mors vers l'avant se traduit par un rapprochement de ceux-ci en vue de réaliser le serrage de l'outil, tandis que le déplacement vers l'arrière des mors s'accompagne d'un desserrage de l'outil.

Un mandrin du type précité est en général équipé d'une pièce centrale qui présente d'une part une partie arrière destinée à être fixée à la machine tournante et sur laquelle est montée une bague arrière, et d'autre part une partie avant à laquelle sont associés les mors, et qui est entourée d'une bague de serrage.

La bague arrière et la bague de serrage ont pour but de permettre à un utilisateur d'actionner le mandrin, par maintien de la bague arrière et actionnement en rotation de la bague de serrage. A cet effet, les mors possèdent une partie filetée, intérieurement ou extérieurement, pouvant être entraînée en rotation soit par un filetage de la pièce centrale, cette dernière étant actionnée par la bague, soit par un écrou périphérique qui est entraîné par la bague de serrage.

Un autre mandrin connu dans l'art est celui du brevet US 4 423 881, qui montre le préambule de la première revendication.

Certaines machines tournantes spécifiques, dites « clé à choc », présentent la particularité d'entraîner en rotation leur broche par un système générant des chocs radiaux sur la broche. Un tel effet permet d'obtenir des puissances de serrage et de desserrage très élevées. Habituellement, les douilles de vissage et autres embouts sont montés directement ou à l'aide d'adaptateurs sur la broche carrée de la machine.

Or, pour certaines applications, il est souhaitable d'équiper la broche de la machine « clé à choc » d'un mandrin sur lequel est fixé l'embout formant outil. Mais sur ce type de machines, un mandrin sans clé classique, même équipé d'un système de verrouillage, s'ouvre immédiatement dans le cas d'une utilisation en rotation à gauche. Ce phénomène est directement lié aux chocs radiaux que génère la machine.

Le but de l'invention est donc de fournir un mandrin du type précité capable d'offrir les fonctionnalités habituelles d'un mandrin, sur ce type de machine, et de ne pas subir de desserrage quel que soit le sens de rotation.

A cet effet, l'invention se réalise avec un mandrin porte-outil pour l'équipement d'une machine tournante, notamment du type «clé à choc» selon la première revendication.

Ainsi, lorsque la pièce centrale tourne dans le premier sens (sens de serrage, généralement à droite), la machine exerce sur le mandrin des chocs radiaux à une fréquence donnée vers la droite. La pièce tournante et la bague sont déjà un contact dans le sens du serrage du mandrin. L'inertie de la bague accentue donc directement l'effet de serrage du mandrin.

A l'inverse, lorsque la pièce centrale tourne dans le deuxième sens (sens de serrage, généralement à gauche), la machine exerce sur le mandrin des chocs radiaux à une fréquence donnée vers la gauche. Compte tenue de son inertie, la bague a tendance à ne pas suivre le mouvement de rotation ainsi généré. En conséquence, les moyens élastiques qui relient la bague à la pièce tournante sont mis en tension, alors que l'angle de battement relatif entre ces deux pièces se réduit. Entre deux chocs générés par la machine, les moyens élastiques ainsi tendus reviennent vers leur position de repos et restituent leur énergie à la bague qui vient alors frapper la pièce tournante dans le sens du serrage, ce qui a pour effet de resserrer les mors et d'éviter toute ouverture intempestive du mandrin.

Selon une réalisation possible, les moyens élastiques sont disposés entre une dent de la bague et une dent de la pièce tournante et, par exemple, relient lesdites dents.

Les moyens élastiques peuvent comprendre un ressort présentant la forme d'une portion de cercle, dont une première extrémité est fixée sur une dent de la bague et dont une deuxième extrémité est fixée sur une dent de la pièce tournante.

Il peut être prévu que la bague et la pièce tournante présentent chacune trois dents sensiblement équiréparties le long de leur face intérieure, respectivement extérieure, la bague étant montée autour de la pièce tournante de sorte que chaque dent de la bague soit placée entre deux dents de la pièce tournante.

Dans ce cas, la deuxième extrémité du ressort peut être fixée sur la dent de la pièce tournante située immédiatement en aval de la dent de la bague sur laquelle la première extrémité du ressort est fixée, par rapport au premier sens de rotation.

Les dents de la bague et de la pièce tournante peuvent présenter chacune une face sensiblement plane et radiale formant surface de butée, lesdites faces étant destinées à coopérer.

Selon une réalisation possible, la bague possède une masse comprise entre 5 et 20 % de la masse totale du mandrin, voire entre 5 et 15 %, voire encore entre 7 et 12 %.

On peut prévoir que la dent de la pièce tournante soit formée par l'interruption d'un évidement circonférentiel ménagé dans ladite pièce tournante.

Selon un premier mode de réalisation, les mors sont guidés en translation dans des logements ménagés dans la pièce centrale formant corps, lesdits mors convergeant vers l'avant et présentant un filetage sur leur face extérieure, et en ce que la pièce tournante est constituée d'un écrou présentant un filetage intérieur coopérant avec le filetage des mors.

Selon un deuxième mode de réalisation, les mors sont guidés en translation dans des logements ménagés dans la pièce tournante formant un corps, les mors convergeant vers l'avant et présentant un filetage sur leur face intérieure, la partie avant de la pièce centrale étant filetée et coopérant avec le filetage des mors.

On décrit à présent, à titre d'exemple non limitatif, une forme de réalisation possible de l'invention, en référence aux figures annexées :

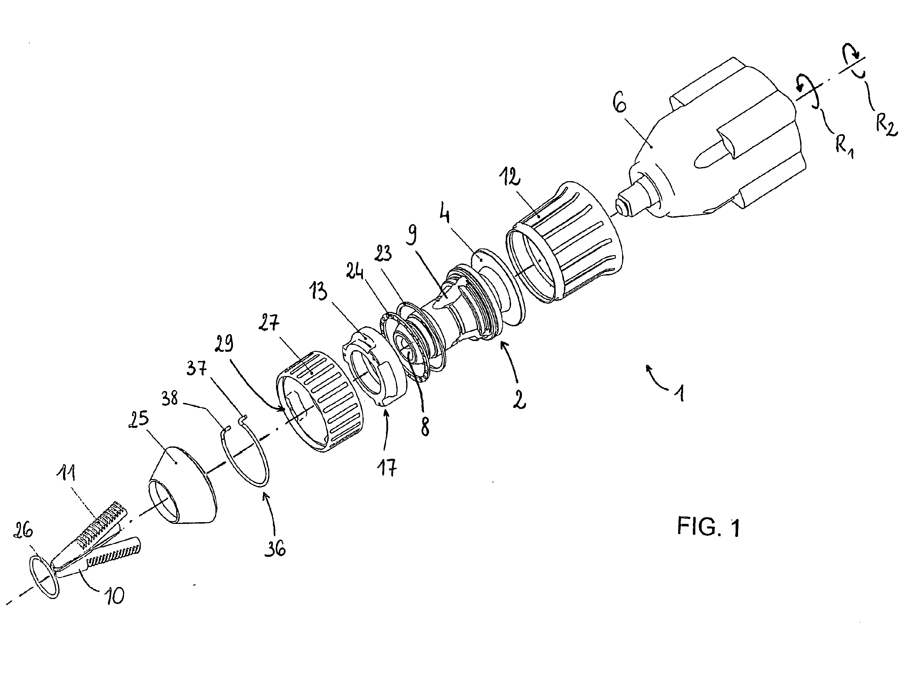

La figure 1 est une vue en perspective éclatée du mandrin selon l'invention ;
La figure 2 en est une vue en coupe longitudinale;
La figure 3 est une vue en perspective de l'écrou;
La figure 4 est une vue en perspective de la bague ;
Les figures 5 et 6 sont des vues en coupe selon la ligne AA de la figure 2, lorsque la pièce centrale tourne à droite, respectivement à gauche.

Le mandrin 1 comprend une pièce centrale formant ici un corps 2, de forme générale cylindrique et d'axe 3. La partie arrière 4 du corps 2 comprend un orifice 5 destiné à permettre l'introduction d'une broche 6 d'une machine tournante telle qu'une perceuse. Dans la réalisation représentée, la broche 6 est de section carrée. La partie avant 7 du corps 2 comprend un alésage 8 longitudinal dans lequel est destiné à être introduit un outil tel qu'un foret, ainsi que trois logements 9 convergeant vers l'avant, recevant chacun un mors 10 et permettant son guidage en translation. Les mors 10 présentent un filetage extérieur 11.

Le mandrin 1 comprend également une bague arrière 12, sensiblement cylindrique, engagée autour de la partie arrière 4 du corps 2 de façon sensiblement coaxiale, et rendue solidaire de celle-ci par tout moyen approprié (par exemple par un système de méplats et l'engagement d'un bourrelet dans une gorge annulaire).

Un écrou 13 est engagé autour des mors 10, sensiblement coaxialement au corps 2. L'écrou 13 présente un filetage intérieur 14 coopérant avec le filetage extérieur 11 des mors 10, pour permettre le déplacement des mors 10 vers la position de serrage ou desserrée suivant le sens d'entraînement en rotation de l'écrou 13.

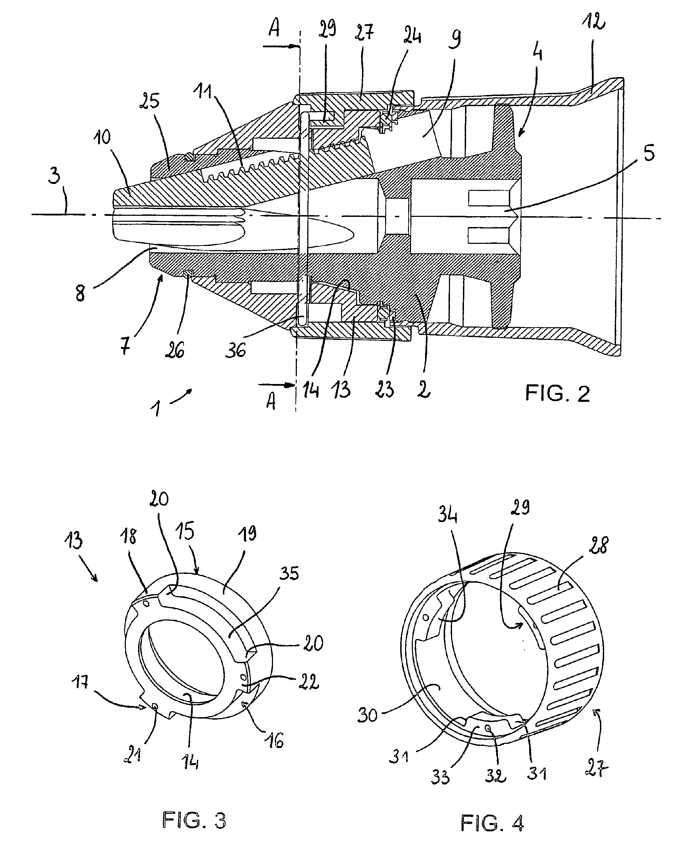

Comme illustré sur la figure 3, l'écrou 13 est sensiblement cylindrique, de longueur faible par rapport à son diamètre et à la longueur du corps 2. L'écrou 13 comprend une partie arrière 15 en forme de couronne et une partie avant présentant trois évidements 16 circonférentiels espacés les uns des autres. Sont ainsi définies trois dents 17, s'étendant sensiblement radialement vers l'extérieur de l'écrou 13, l'extrémité libre 18 des dents 17 étant située dans le prolongement de la partie arrière 15 de l'écrou 13, et formant avec elle la face extérieure 19 de l'écrou 13. Les dents 17 sont équiréparties à la périphérie de l'écrou 13. Chaque dent 17 est délimitée par deux faces radiales 20, et couvre un angle d'environ 20 à 30°. De plus, chaque dent 17 comprend un orifice 21 pratiqué sensiblement axialement depuis sa face avant 22, laquelle est orthogonale à l'axe de l'écrou 13.

L'écrou 13 est monté en butée arrière sur le corps 2. Afin de faciliter sa rotation, une rondelle en acier 23 et une cage à billes 24 sont interposées entre l'écrou 13 et le corps 2.

Un nez métallique 25 est monté sur le corps 2 en avant de l'écrou 13, permettant ainsi de maintenir axialement l'écrou 13. Le nez métallique 25 est lui-même bloqué axialement vers l'avant par un jonc 26 engagé dans une gorge de la partie avant 7 du corps 2.

Le mandrin 1 comprend également une bague 27 servant au serrage, sensiblement cylindrique, montée autour de l'écrou 13 sensiblement coaxialement, et bloquée en rotation vers l'arrière par la bague arrière 12 et vers l'avant par le nez métallique 25. La bague 27 présente un moletage 28 sur sa face extérieure destiné à faciliter sa manipulation par un utilisateur.

La bague 27 comprend, en partie avant, trois dents 29 faisant saillie de sa face intérieure 30 sensiblement radialement et vers l'intérieur. Les dents 29 sont sensiblement équiréparties de façon circonférentielle le long de la face intérieure 30. Chaque dent 29 est délimitée par deux faces radiales 31 et couvre un angle d'environ 40 à 50°. De plus, chaque dent 29 comprend un orifice 32 pratiqué sensiblement axialement depuis sa face avant 33, laquelle est orthogonale à l'axe de la bague 27.

La bague 27 est montée autour de l'écrou 13 de sorte que chacune de ses dents 29 soit placée entre deux dents 17 de l'écrou 13. La hauteur des dents 29 est telle que, lorsque la bague 27 est montée autour de l'écrou 13, l'extrémité libre 34 d'une dent 29 est située à proximité, mais avec jeu, du fond 35 de l'évidement 16 correspondant. Dans cette position montée, l'extrémité libre 18 des dents 17 est située à proximité, mais avec jeu, de la face intérieure 30 de la bague 27.

La bague 27 présente un moment d'inertie important par rapport à l'axe 3 du corps 2, obtenu par une masse importante. A titre d'exemple, la masse de la bague 27 est comprise entre 40 et 50 g pour une masse totale du mandrin 1 de l'ordre de 400 g.

Enfin, le mandrin 1 comprend un ressort 36 en forme de portion de cercle, couvrant par exemple un angle compris entre 250 et 300°. Une première extrémité 37 du ressort 36 est fixée dans l'orifice 32 d'une dent 29 de la bague 27, et la deuxième extrémité 38 du ressort 36 est fixée dans l'orifice 21 d'une dent 17 de l'écrou 13 située immédiatement en aval par rapport au sens de serrage.

Pour le serrage ou le desserrage d'un outil dans l'alésage 8 du corps 2 du mandrin 1, un utilisateur tient la bague arrière 12 d'une main et, avec l'autre main, actionne la bague 27 en rotation par rapport à la bague arrière 12. Ainsi, il provoque la rotation de l'écrou 13 du fait de la coopération entre les dents 17 et 29. En conséquence, par la coopération entre le filetage extérieur 11 des mors 10 et le filetage intérieur 14 de l'écrou 13, les mors 10 sont déplacés dans les logements 9 du corps 2. Selon le sens de rotation de la bague 27, les mors 10 sont déplacés vers leur position de serrage, où ils sont saillants vers l'avant du mandrin 1 et rapprochés les uns des autres de façon à pouvoir maintenir l'outil serré entre eux, ou vers une position desserrée, où ils sont rétractés au moins partiellement à l'intérieur du mandrin 1 et écartés les uns des autres, l'outil pouvant alors être ôté du mandrin 1.

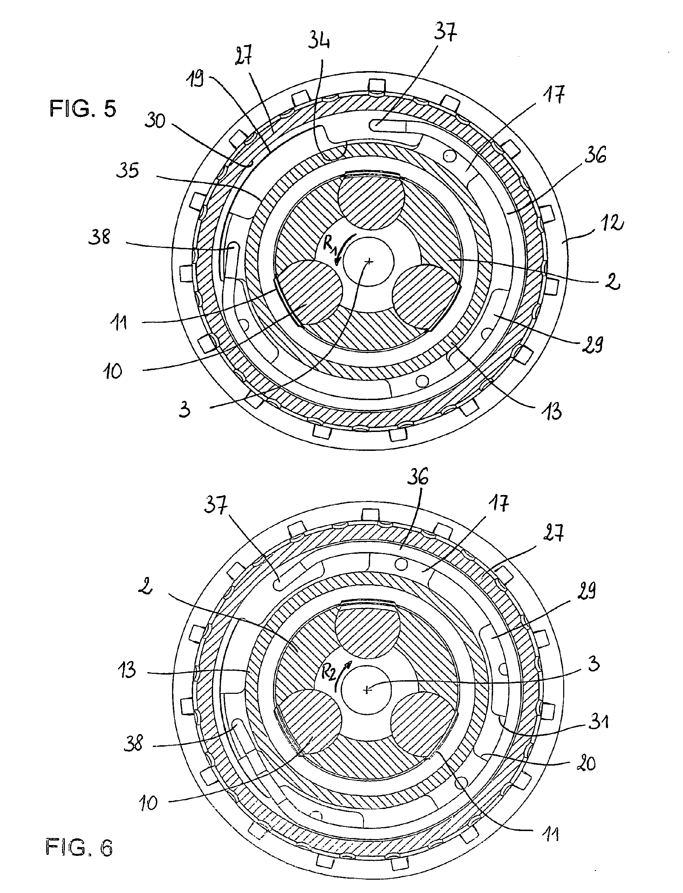

On se réfère à présent aux figures 5 et 6 qui illustrent la position relative des différentes pièces constitutives du mandrin 1 selon le sens de rotation de la machine tournante.

Lorsque la broche 6 tourne à droite, ce qui correspond au sens de serrage, elle entraîne le corps 2 en rotation à droite (figure 5, flèche R1), ainsi que l'écrou 13 via les mors 10. La face radiale 20 aval (par rapport au sens R1) de chaque dent 17 de l'écrou 13 est en contact avec la face radiale 31 amont d'une dent 29 de la bague 27. En conséquence, la rotation à droite de l'écrou 13 entraîne la rotation à droite de la bague 27. Dans cette configuration, le ressort 36 est en position de repos ou légèrement tendu.

Lorsque la broche 6 tourne à gauche, ce qui correspond au sens de desserrage, elle entraîne le corps 2 en rotation à gauche (figure 6, flèche R2) et, par conséquent, les mors 10 engagés dans les logements 9 du corps 2. Par coopération entre les filetages intérieur 14 de l'écrou 13 et extérieur 11 des mors 10, l'écrou 13 amorce une rotation à gauche qui ne peut se poursuivre au-delà d'un certain angle de rotation du fait du desserrage progressif des mors 10.

Puisqu'il existe un écart angulaire entre la face radiale 20 aval (par rapport au sens R2) de chaque dent 17 de l'écrou 13 et la face radiale 31 amont de la dent 29 correspondante de la bague 27, l'écrou 13 ne peut entraîner la bague 27 par l'intermédiaire de la coopération directe entre les dents. En outre, du fait de l'importante inertie en rotation de la bague 27, le ressort 36, dont la deuxième extrémité est entraînée en rotation selon R2 par la dent 17 de l'écrou 13 à laquelle il est fixé, ne permet pas non plus un entraînement simultané de la bague 27 et de l'écrou 13.

La bague 27 ne suivant pas la rotation à gauche de l'écrou 13, l'écart angulaire entre les dents 17, 29 qui étaient en contact dans le cas de la rotation à droite (R1) augmente, et le ressort 36 se tend, emmagasinant ainsi l'énergie qui servira au resserrage.

Pendant le moment où le mouvement à gauche de la machine tournante est arrêté (fonctionnement par chocs radiaux), et sous l'effet de la force de rappel du ressort 36, la bague 27 est brusquement entraînée en rotation à gauche. Ses dents 29 « rattrapent » les dents 17 de l'écrou 13 qu'elles viennent heurter avec un choc qui provoque la rotation de l'écrou 13 vers la gauche par rapport au corps 2, et donc le resserrage des mors 10. Les pièces constitutives du mandrin 1 se retrouvent alors dans la position de la figure 5.

L'homme du métier comprendra que la masse de la bague 27 (ou plus généralement son moment d'inertie par rapport à l'axe 3 du corps 2), la raideur du ressort 36 et le frottement entre la bague 27 et l'écrou 13 doivent être judicieusement choisis pour permettre de provoquer un retard suffisant en rotation de la bague 27 par rapport à l'écrou 13 et un choc suffisant des dents 29 sur les dents 17 pour permettre le resserrage des mors 10.

Il va de soi que l'invention n'est pas limitée à la forme de réalisation décrite ci-dessus à titre d'exemple mais qu'elle en embrasse au contraire toutes les variantes de réalisation qui tombent au sein de la première revendication.

En variante, les moyens élastiques pourraient par exemple, être formés d'une mousse compressible disposée entre deux dents adjacentes de la bague et de la pièce tournante.

## Revendications

1. Mandrin porte-outil pour l'équipement d'une machine tournante, notamment du type « clé à choc », comprenant :
- une pièce centrale (2) présentant un axe (3) et comportant une partie arrière (4) destinée à être fixée sur un arbre moteur de la machine tournante et une partie avant (7) à laquelle sont associés des mors (10), lesdits mors (10) pouvant être déplacés par rapport à la pièce centrale (2) entre une position de serrage de l'outil et une position desserrée ;
- une pièce tournante (13) qui, placée autour de la pièce centrale (2) et associée aux mors (10), est destinée à réaliser le déplacement des mors (10) en vue du serrage ou du desserrage de l'outil, ladite pièce tournante (13) étant, en période de fonctionnement, solidaire en rotation de la pièce centrale (2) lorsque celle dernière tourne dans un premier sens (R1), correspondant au sens de serrage ;
- une bague (27) montée autour de la pièce tournante (13) en vue de réaliser l'entraînement de ladite pièce tournante (13)
- la bague (27) étant montée autour de la pièce tournante (13) de façon mobile en rotation selon une amplitude angulaire limitée ;
caractérisé:
- en ce que la pièce tournante (13) comprend au moins une dent (17) s'étendant sensiblement radialement vers l'extérieur, destinée à coopérer avec une dent (29) faisant saillie de la bague (27) sensiblement radialement vers l'intérieur, pour pouvoir entraîner la bague (27) en rotation dans le premier sens (R1);
- et en ce que des moyens élastiques (36) sont disposés entre la pièce tournante (13) et la bague (27), la bague (27) présentant un moment d'inertie suffisamment important par rapport à l'axe (3) de la pièce centrale (2) pour que, lorsque la pièce centrale (2) tourne dans un deuxième sens (R2), correspondant au sens de desserrage, la bague (27) ne suive pas initialement le mouvement de rotation dans le deuxième sens (R2) amorcé par la pièce tournante (13), provoquant ainsi la tension ou la compression des moyens élastiques (36) puis, sous l'effet de la force de rappel des moyens élastiques (36), la dent (29) de la bague (27) vienne heurter la dent (17) de la pièce tournante (13) en provoquant le serrage des mors (10).

2. Mandrin selon la revendication 1, **caractérisé en ce que** les moyens élastiques (36) sont disposés entre une dent (29) de la bague (27) et une dent (17) de la pièce tournante (13).

3. Mandrin selon la revendication 1 ou 2, **caractérisé en ce que** les moyens élastiques (36) relient une dent (29) de la bague (27) et une dent (17) de la pièce tournante (13).

4. Mandrin selon l'une des revendications 1 à 3, **caractérisé en ce que** les moyens élastiques comprennent un ressort (36) présentant la forme d'une portion de cercle, dont une première extrémité (37) est fixée sur une dent (29) de la bague (27) et dont une deuxième extrémité (38) est fixée sur une dent (17) de la pièce tournante (13).

5. Mandrin selon l'une des revendications 1 à 4, **caractérisé en ce que** la bague (27) et la pièce tournante (13) présentent chacune trois dents (29, 17) sensiblement équiréparties le long de leur face intérieure (30), respectivement extérieure (19), la bague (27) étant montée autour de la pièce tournante (13) de sorte que chaque dent (29) de la bague (27) soit placée entre deux dents (17) de la pièce tournante (13).

6. Mandrin selon les revendications 4 et 5, **caractérisé en ce que** la deuxième extrémité (38) du ressort (36) est fixée sur la dent (17) de la pièce tournante (13) située immédiatement en aval de la dent (29) de la bague (27) sur laquelle la première extrémité (37) du ressort (36) est fixée, par rapport au premier sens de rotation (R1).

7. Mandrin selon l'une des revendications 1 à 6, **caractérisé en ce que** les dents (29, 17) de la bague (27) et de la pièce tournante (13) présentent chacune une face sensiblement plane et radiale formant surface de butée, lesdites faces (31, 20) étant destinées à coopérer.

8. Mandrin selon l'une des revendications 1 à 7, **caractérisé en ce que** la bague (27) possède une masse comprise entre 5 et 20 % de la masse totale du mandrin (1).

9. Mandrin selon l'une des revendications 1 à 8, **caractérisé en ce que** la dent (17) de la pièce tournante (13) est formée par l'interruption d'un évidement (16) circonférentiel ménagé dans ladite pièce tournante (13).

10. Mandrin selon l'une des revendications 1 à 9, **caractérisé en ce que** les mors (10) sont guidés en translation dans des logements (9) ménagés dans la pièce centrale formant corps (2), lesdits mors (10) convergeant vers l'avant et présentant un filetage (11) sur leur face extérieure, et **en ce que** la pièce tournante est constituée d'un écrou (13) présentant un filetage intérieur (14) coopérant avec le filetage (11) des mors (10).

11. Mandrin selon l'une des revendications 1 à 9, **caractérisé en ce que** les mors (10) sont guidés en translation dans des logements ménagés dans la pièce tournante (13) formant un corps, les mors (10) convergeant vers l'avant et présentant un filetage sur leur face intérieure, la partie avant de la pièce centrale (2) étant filetée et coopérant avec le filetage des mors (10).

## Claims

1. A tool holder chuck for equipping a rotating machine, notably of the « impact key » type comprising:
- a central part (2) having an axis (3) and including a rear portion (4) intended to be attached on a driving spindle of the rotating machine and a front portion (7) with which jaws (10) are associated, said jaws (10) being displaceable relatively to the central part (2) between a position for tightening the tool and a loosening position;
- a rotating part (13) which, placed around the central part (2), and associated with the jaws (10), is intended to produce the displacement of the jaws (10), with view to tightening or loosening the tool, said rotating part (13) being, during operation, interdependent in rotation with the central part (2) when the latter rotates in a first direction (R1), corresponding to the tightening direction;
- a ring (27) mounted around the rotating part (13), with view to achieving driving of said rotating part (13),
- the ring (27) being mounted around the rotating part (13) so as to be rotationally mobile along a limited angular amplitude;
**characterized:**
- **in that** the rotating part (13) comprises at least one tooth (17), extending substantially radially outwards, intended to cooperate with a tooth (29) protruding from the ring (27) substantially radially inwards, in order to be able to drive the ring (27) into rotation in the first direction (R1);
- and **in that** elastic means (36) are positioned between the rotating part (13) and the ring (27), the ring (27) having a sufficiently large moment of inertia relatively to the axis (3) of the central part (2) so that, when the central part (2) rotates in a second direction (R2), corresponding to the loosening direction, the ring (27) does not initially follow the rotary movement in the second direction (R2) initiated by the rotating part (13), thereby causing tensioning or compression of the elastic means (36) and then, under the effect of the return force of the elastic means (36), the tooth (29) of the ring (27) will hit the tooth (17) of the rotating part (13) causing tightening of the jaws (10).

2. The chuck according to claim 1, **characterized in that** the elastic means (36) are positioned between a tooth (29) of the ring (27) and a tooth (17) of the rotating part (13).

3. The chuck according to claim 1 or 2, **characterized in that** the elastic means (36) connect a tooth (29) of the ring (27) and a tooth (17) of the rotating part (13).

4. The chuck according to one of claims 1 to 3, **characterized in that** the elastic means comprise a spring (36) having the shape of a circular portion, a first end (37) of which is attached on a tooth (29) of the ring (27) and a second end (38) of which is attached on a tooth (17) of the rotating part (13).

5. The chuck according to one of claims 1 to 4, **characterized in that** the ring (27) and the rotating part (13) each have three teeth (29, 17) substantially equally distributed along their inner (30), respectively outer (19) face, the ring (27) being mounted around the rotating part (13) so that each tooth (29) of the ring (27) is placed between two teeth (17) of the rotating part (13).

6. The chuck according to claims 4 and 5, **characterized in that** the second end (38) of the spring (36) is attached on the tooth (17) of the rotating part (13) immediately located downstream from the tooth (29) of the ring (27) on which the first end (37) of the spring (36) is attached, relatively to the first direction of rotation (R1).

7. The chuck according to one of claims 1 to 6, **characterized in that** the teeth (29, 17) of the ring (27) and of the rotating part (13) each have a substantially planar and radial face forming an abutment surface, said faces (31, 20) being intended to cooperate.

8. The chuck according to one of claims 1 to 7, **characterized in that** the ring (27) has a mass is comprised between 5 and 20% of the total mass of the chuck (1).

9. The chuck according to one of claims 1 to 8, **characterized in that** the tooth (17) of the rotary part (13) is formed by interruption of a circumferential recess (16) made in said rotating part (13).

10. The chuck according to one of claims 1 to 9, **characterized in that** the jaws (10) are guided in translation in housings (9) made in the body-forming central part (2), said jaws (10) converging forwards and having a thread (11) on their outer face, and **in that** the rotating part consists of a nut (13) having an inner thread (14) cooperating with the thread (11) of the jaws (10).

11. The chuck according to one of claims 1 to 9, **characterized in that** the jaws (10) are guided in translation in housings made in the body-forming rotating part (13), the jaws (10) converging forwards and having a thread on their outer face, the front portion of the central part (2) being threaded and cooperating with the thread of the jaws (10).

## Patentansprüche

1. Werkzeughaltefutter zur Ausstattung einer drehenden Maschine, insbesondere vom Typ Drehschlagschrauber, das umfasst:
- ein zentrales Teil (2), das eine Achse (3) aufweist und einen hinteren Abschnitt (4), der dazu bestimmt ist, auf einer Antriebswelle der drehenden Maschine befestigt zu sein, und einen vorderen Abschnitt (7), dem Backen (10) zugeordnet sind, aufweist, wobei die Backen (10) im Verhältnis zum zentralen Teil (2) zwischen einer Spannstellung des Werkzeugs und einer gelösten Stellung verlagerbar sind,
- ein drehendes Teil (13), das, um das zentrale Teil (2) platziert und mit den Backen (10) verbunden, dazu bestimmt ist, die Verlagerung der Backen (10) im Hinblick auf das Spannen oder Lösen des Werkzeugs durchzuführen, wobei das drehende Teil (13) im Betriebszeitraum, mit dem zentralen Teil (2) drehend verbunden ist, wenn dieses Letztere in eine erste Richtung (R1) dreht, die der Spannrichtung entspricht,
- einen Ring (27), der um das drehende Teil (13) montiert ist, um den Antrieb des drehenden Teils (13) durchzuführen,
- wobei der Ring (27) gemäß einer begrenzten Winkelamplitude rotierend bewegbar um das drehende Teil (13) montiert ist,
- **dadurch gekennzeichnet, dass** das drehende Teil (13) mindestens einen Zahn (17) umfasst, der sich etwa radial nach außen erstreckt, der dazu bestimmt ist, mit einem Zahn (29) zusammenzuarbeiten, der aus dem Ring (27) etwa radial nach innen hervorsteht, um den Ring (27) rotierend in die erste Richtung (R1) antreiben zu können,
- und dass elastische Mittel (36) zwischen dem drehenden Teil (13) und dem Ring (27) angeordnet sind, wobei der Ring (27) ein Trägheitsmoment aufweist, das im Verhältnis zur Achse (3) des zentralen Teil (2) ausreichend stark ist, damit, wenn das zentrale Teil (2) in eine zweite Richtung (R2) dreht, die der Löserichtung entspricht, der Ring (27) die Rotationsbewegung in die zweite Richtung (R2), die von dem drehenden Teil (13) angestoßen wird, anfänglich nicht mitmacht, wodurch die Spannung oder die Kompression der elastischen Mittel (36) hervorgerufen wird und dann, unter der Wirkung der Rückholkraft der elastischen Mittel (36), der Zahn (29) des Rings (27), indem das Spannen der Backen (10) verursacht wird, an den Zahn (17) des drehenden Teils (13) anstößt.

2. Futter nach Anspruch 1, **dadurch gekennzeichnet, dass** die elastischen Mittel (36) zwischen einem Zahn (29) des Rings (27) und einem Zahn (17) des drehenden Teils (13) angeordnet sind.

3. Futter nach Anspruch 1 oder 2, **dadurch gekennzeichnet, dass** die elastischen Mittel (36) einen Zahn (29) des Rings (27) und einen Zahn (17) des drehenden Teils (13) verbinden.

4. Futter nach einem der Ansprüche 1 bis 3, **dadurch gekennzeichnet, dass** die elastischen Mittel eine Feder (36) umfassen, die die Form eines Kreisabschnitts aufweist, von der ein erstes Ende (37) auf einem Zahn (29) des Ring (27) befestigt und von der ein zweites Ende (38) auf einem Zahn (17) des drehenden Teils (13) befestigt ist.

5. Futter nach einem der Ansprüche 1 bis 4, **dadurch gekennzeichnet, dass** der Ring (27) und das drehende Teil (13) jeweils drei Zähne (29, 17) aufweisen, die etwa gleichmäßig entlang ihrer Innenseite (30) beziehungsweise Außenseite (19) verteilt sind, wobei der Ring (27) um das drehende Teil (13) derart montiert ist, dass jeder Zahn (29) des Rings (27) zwischen zwei Zähnen (17) des drehenden Teils (13) platziert ist.

6. Futter nach den Ansprüchen 4 und 5, **dadurch gekennzeichnet, dass** das zweite Ende (38) der Feder (36) auf dem Zahn (17) des drehenden Teils (13) befestigt ist, der sich unmittelbar nach dem den Zahn (29) des Rings (27) befindet, auf dem das erste Ende (37) der Feder (36) im Verhältnis zur ersten Rotationsrichtung (R1) befestigt ist.

7. Futter nach einem der Ansprüche 1 bis 6, **dadurch gekennzeichnet, dass** die Zähne (29, 17) des Rings (27) und des drehenden Teils (13) jeweils eine etwa ebene und radiale Fläche aufweisen, die eine Anschlagfläche bilden, wobei die Flächen (31, 20) zur Zusammenarbeit bestimmt sind.

8. Futter nach einem der Ansprüche 1 bis 7, **dadurch gekennzeichnet, dass** der Ring (27) eine Masse zwischen 5 und 20 % inklusive der Gesamtmasse des Futters (1) besitzt.

9. Futter nach einem der Ansprüche 1 bis 8, **dadurch gekennzeichnet, dass** der Zahn (17) des drehenden Teils (13) durch die Unterbrechung einer Umfangsaussparung (16) gebildet ist, die in das drehende Teil (13) eingearbeitet ist.

10. Futter nach einem der Ansprüche 1 bis 9, **dadurch gekennzeichnet, dass** die Backen (10) verschiebend in Aufnahmen (9) geführt sind, die in das zentrale Teil eingearbeitet sind, das den Körper (2) bildet, wobei die Backen (10) nach vorn zusammenlaufen und ein Gewinde (11) auf ihrer Außenseite aufweisen, und dass das drehende Teil von einer Mutter (13) gebildet wird, die ein Innengewinde (14) aufweist, das mit dem Gewinde (11) der Backen (10) zusammenarbeitet.

11. Futter nach einem der Ansprüche 1 bis 9, **dadurch gekennzeichnet, dass** die Backen (10) verschiebend in Aufnahmen (9) geführt sind, die in das drehende Teil (13) eingearbeitet sind, das einen Körper bildet, wobei die Backen (10) nach vorn zusammenlaufen und ein Gewinde auf ihrer Innenseite aufweisen, wobei der vordere Abschnitt des zentralen Teils (2) ein Gewinde hat und mit dem Gewinde der Backen (10) zusammenarbeitet.
